# EUROPEAN PATENT SPECIFICATION

(11) **EP 3 355 969 B1**
(45) Date of publication and mention of the grant of the patent: **20.11.2019**
(21) Application number: 16787348.8
(22) Date of filing: 30.09.2016
(51) Int. Cl.: A61M 5/315, A61M 5/20

(54) **PISTON WASHER ASSEMBLY, METHOD OF ASSEMBLY AND DRUG DELIVERY DEVICE INCORPORATING SUCH PISTON WASHER ASSEMBLY**
KOLBENUNTERLEGSCHEIBENANORDNUNG, MONTAGEVERFAHREN UND ARZNEIMITTELABGABEVORRICHTUNG MIT SOLCH EINER KOLBENUNTERLEGSCHEIBENANORDNUNG
ENSEMBLE RONDELLE DE PISTON, PROCÉDÉ D'ASSEMBLAGE ET DISPOSITIF D'ADMINISTRATION DE MÉDICAMENT INCORPORANT UN TEL ENSEMBLE RONDELLE DE PISTON

(30) Priority: 01.10.2015 EP 15188025
(43) Date of publication of application: 08.08.2018
(73) Proprietor: Novo Nordisk A/S, 2880 Bagsværd (DK)
(72) Inventor: BENGTSSON, Henrik, 2800 Bagsværd (DK)
(86) International application number: PCT/EP2016/073480
(87) International publication number: WO 2017/055588

(56) References cited:
- WO-A1-2011/042539
- WO-A1-2012/143494
- JP-A- 2007 267 906
- JP-A- 2007 289 676

## Description

The present invention relates to an assembly of components for a drug delivery device that allows a user to select single or multiple doses of an injectable liquid drug and to dispense the set dose of the product and to apply said product to a patient, preferably by injection. In particular, the present invention relates to a piston washer assembly and method of forming such piston washer assembly.

### BACKGROUND

In the disclosure of the present invention reference is mostly made to drug delivery devices used e.g. in the treatment of diabetes by delivery of insulin, however, this is only an exemplary use of the present invention.

Drug delivery devices allowing for multiple dosing of a required dosage of a liquid medicinal product, such as liquid drugs, and further providing administration of the liquid to a patient, are as such well-known in the art. Generally, such devices have substantially the same purpose as that of an ordinary syringe. Drug delivery devices of this kind have to meet a number of user specific requirements. For instance in case of those with diabetes, many users will be physically infirm and may also have impaired vision. Therefore, these devices need to be robust in construction, yet easy to use, both in terms of the manipulation of the parts and understanding by a user of its operation. Further, the dose setting must be easy and unambiguous and where the device is to be disposable rather than reusable, the device should be inexpensive to manufacture and easy to dispose. In order to meet these requirements, the number of parts and steps required to assemble the device and an overall number of material types the device is made from have to be kept to a minimum.

Typically, the liquid drug to be administered is provided in a cartridge that has a moveable piston or bung mechanically interacting with a piston rod of an expelling mechanism of the drug delivery device. By applying thrust to the piston in distal direction, a predefined amount of the liquid drug is expelled from the cartridge. Due to inevitable manufacturing tolerances there may for instance persist axial clearance between a cartridge's piston and the piston rod. Typically, prior to a primary use of the device, an end-user has to conduct a so-called priming of the expelling mechanism in order to ensure, that already with an initial dose setting and a first subsequent dose dispensing step, an accurate amount of the liquid drug is dispensed in a predefined way. An initial dose setting and expelling of a minor dose may in certain situations also be required for removing any air present in the cartridge and/or a connected needle and for performing a flow check.

Document WO 99/38554 A1 discloses several embodiments of injection devices each suitable for forming a disposable device wherein a liquid drug cartridge is inserted into the device during assembly in a production line.

State of the art pen-type drug delivery devices that incorporate a dose setting feature often include a so-called end-of-content limiter to prevent a user from selecting a size of a dose which exceeds the amount of liquid drug remaining in a cartridge of the device. References WO 01/19434 A1, WO 2006/128794 A2 and WO 2010/149209 A1 include disclosure of such end-of-content limiters.

In the production line, during final assembly operations of the devices, at least a part of the priming is typically carried out using the dose setting and expelling mechanism so that users will experience virtually consistent requirement for a priming operation across individual pen samples irrespective of the initial gap between the piston rod and the piston which emanates from tolerances. References WO 2009/095332 A1 and WO2011/121061 A1 disclose devices wherein a distance between the distal end of a piston rod means and the piston is minimized or reduced to zero. However, the solutions are not readily suitable for use where a piston rod is rotated during dose expelling. Further, for eliminating clearance between piston and piston rod, reference WO 2011/051365 A2 suggests to select and insert at least one spacer from a set of differently sized spacers. However, in high volume production, the use of spacers with only minute differences in dimensions introduces the risk of spacers of different sizes being mixed up WO2011/042539 shows a device having a piston washer assembly.

### SUMMARY

It is an object of the present invention to provide a drug delivery device featuring improved and facilitated clearance reduction or clearance elimination. It is a further object of the invention to provide a simplified and robust method of eliminating clearance in a drug delivery device. Finally, it is an object of the invention to provide manufacture of drug delivery devices providing consistently uniform and predictable total doseable amount of liquid drug from a held cartridge.

In the disclosure of the present invention, embodiments and aspects will be described which will address one or more of the above objects or which will address objects apparent from the below disclosure as well as from the description of exemplary embodiments.

In a first aspect, the present invention relates to a piston washer assembly for use in a drug delivery device for transferring a distally directed axial force from a piston rod towards a piston of a held cartridge. The piston washer assembly comprises:
- a proximal part adapted for engagement with the distal portion of a piston rod, the proximal part defining a first set of cooperating geometries, and
- a distal part configured to engage and abut a piston of a held cartridge and to engage the proximal part, wherein the distal part defines a second set of cooperating geometries engaging the first set of cooperating geometries.

In an initial state of the piston washer assembly, the proximal part and the distal part are positioned relatively to each other to define an initial axial dimension (H, H1) for the piston washer assembly, and wherein the proximal part and the distal part are movable relative to each other by moving the first set of cooperating geometries and the second set of cooperating geometries relative to each other to increase said axial dimension (H) until the proximal part and the distal part assume a target state where said axial dimension (H) reaches an axial target dimension (H2).

In some embodiments, the first set of cooperating geometries and the second set of cooperating geometries are configured to prevent the distal part and the proximal part from being moved relative to each other when forces act on the proximal part and the distal part to decrease said axial target dimension.

In some embodiments the first set of cooperating geometries and the second set of cooperating geometries are configured to provide a detent mechanism allowing the axial dimension (H) to be increased while preventing the axial dimension (H) to be decreased. In some embodiments, the detent mechanism defines a one-way detent mechanism.

The first set of cooperating geometries and the second set of cooperating geometries may define a one-way incremental detent mechanism allowing the axial dimension (H) to be increased in incremental steps of unit size ΔH. When the proximal part and the distal part assume the target state, the first set of cooperating geometries and the second set of cooperating geometries prevent substantial movement of the first part and the second part relative to each other acting to reduce the axial dimension (H), i.e. a backwards movement. A slight backwards movement may be assumed, in particular if the target axial dimension (H2) is a little less than a state wherein a full incremental step is allowed, i.e. if the target axial dimension (H2) falls between two consecutive steps. However such backwards movement will always be less than the magnitude of the unit step ΔH.

In some embodiments the first set of cooperating geometries defines a first thread configuration and the second set of cooperating geometries defines a second thread configuration engaging the first thread configuration. The relative rotational movement in a first rotational direction between the proximal part and the distal part will cause an increase in the axial dimension of the piston washer assembly.

In particular embodiments, an incremental positioning mechanism may be arranged between the proximal part and the distal part causing said relative rotational movement between the proximal part and the distal part to occur in incremental angular steps.

The incremental positioning mechanism may be provided as cooperating detent geometries arranged in the first thread configuration and the second thread configuration. In other embodiments, the detent geometries are arranged at other locations outside the thread configurations. The thread configurations may be provided as continuous threads or as a plurality of individual thread segments. The lead of the thread configuration may be constant or may be of varying lead along the threaded configuration.

In other embodiments the first set of cooperating geometries and the second set of cooperating geometries define a linear guide allowing linear axial movement between the proximal part and the distal part. The first set of cooperating geometries and the second set of cooperating geometries may define a toothed one-way ratchet engagement allowing the axial dimension (H) of the piston washer assembly to be increased but preventing the axial dimension (H) to be decreased.

In particular embodiments the piston washer assembly defines a peripheral section having a diameter of a first magnitude and wherein the maximum settable axial dimension is smaller than the first magnitude.

The piston washer assembly may be configured for being loosely withheld between the piston rod of the drug delivery device and the piston of a held cartridge. Hence, when the piston washer assembly assumes its final configuration in a drug delivery device, the piston rod will be able to rotate independently from the piston washer assembly, i.e. allowing the piston washer to be kept non-rotatable while the piston rod rotates, e.g. during dose expelling.

In certain embodiments said axial dimension defines the axial distance between a central proximal surface portion of the proximal part and a peripheral distal surface portion of the distal part. The distal part of the piston washer assembly may be formed exhibiting a generally cylindrical peripheral outer surface. Also the proximal part of the piston washer assembly may be formed as a member exhibiting a general cylindrical outer surface.

The proximal facing surface of the proximal part may comprise a recessed central area having an annular surface area that act as a bearing surface for engagement with a convex annular surface of the piston rod allowing low-friction rotational movement of the piston rod relative to the proximal part of the piston washer assembly. In other embodiments, the proximal facing surface of the proximal part may comprise a protruding central area having an annular surface area that act as a bearing surface for engagement with a concave annular surface of the piston rod. Said central area of the proximal part may include an annular surface which may be formed exhibiting a conical or part-spherical surface.

In a second aspect, the present invention relates to a drug delivery device incorporating a piston washer in accordance with the first aspect.

The drug delivery device may define a device for expelling a dose of drug from a held cartridge, the drug delivery device defining a distal drug outlet end and an opposite proximal end and comprising:
- a housing component holding a cartridge comprising a liquid drug and a piston slideable arranged therein in an axial direction,
- a dose setting and expelling mechanism coupled to the housing component and comprising a piston rod for exerting a force on the piston of the cartridge in a distal direction for expelling a dose, and
wherein a piston washer assembly according to any variants identified above in connection with the first aspect is arranged axially between the piston of the cartridge and the piston rod.

The piston rod of the drug delivery device may be configured for being rotated during dose expelling such as when a thread of the piston rod engages with a threaded nut arranged at a fixed position within the housing. The piston rod may be rotatably arranged relative to the piston washer assembly to thereby allow the piston rod to rotate while the piston washer assembly remains non-rotatable during dose expelling.

The dose setting and expelling mechanism may comprise a dose setting element that rotates relative to a driver during setting of a dose and wherein the dose setting element does not rotate relative to the driver during expelling of a set dose.

In a third aspect of the invention an end-of-content limiter may be arranged between the driver and the dose setting element of the drug delivery device according to the second aspect. The end-of-content limiter may be configured to prevent setting of a dose which exceeds a doseable amount of liquid drug remaining in the cartridge.

In exemplary embodiments the end-of-content limiter is engaging the driver and the dose setting element. The end-of-content limiter moves towards an end-of-content stop geometry for example provided by one of the driver and the dose setting element as the dose setting element is rotated relative to the driver for dialling up a dose.

In a fourth aspect, the invention relates to a method of preparing a piston washer assembly as defined above and for use in a drug delivery device for transferring a distally directed axial force from a piston rod towards a piston of a held cartridge. The method comprises the steps of:
- providing the proximal part and the distal part in an initially engaged state, wherein the proximal part and the distal part are positioned relatively to each other to define an initial axial dimension for the piston washer assembly,
- estimating a desired axial target dimension for the assembly, and
- moving the proximal part and the distal part relative to each other to increase said axial dimension until the proximal part and the distal part assume a target state where said axial dimension reaches the desired axial target dimension.

The method may further comprise the steps of:
- providing one of the distal part and the proximal part as a first injection shot in a multi-shot injection molding process, and
- providing the other of the distal part and the proximal part as a further injection shot in a multi-shot injection molding process so that the first set of cooperating geometries and the second set of cooperating geometries at least partly engage.

The method step of moving the proximal part and the distal part relative to each other to increase said axial dimension until the proximal part and the distal part assume a target state where said axial dimension reaches the desired axial target dimension may in certain embodiments comprise the steps of:
- maintaining the distal part engaged with a first tool part,
- maintaining the proximal part engaged with a second tool part,
- moving the first tool part and the second tool part relative to each other while measuring, determining or estimating the size of said axial dimension of the piston washer assembly.

Finally, in a fifth aspect, the present invention relates to a method of assembling a drug delivery device as defined in accordance with the second aspect, comprising the steps of:
- positioning the cartridge comprising a liquid drug and a piston slideably arranged therein in a first housing component to form a first sub-assembly,
- positioning the dose setting and expelling mechanism comprising the piston rod in a second housing component to form a second sub-assembly,
- determining the axial position of the piston with respect to the first housing component,
- determining the axial position of the piston rod with respect to the second housing component,
- determining or estimating the size of axial clearance between the piston rod and the piston if the first housing component and the second housing component were assembled and selecting a target axial dimension (H2) for a piston washer assembly to be positioned between the piston rod and the piston in accordance with said size of axial clearance,
- modifying the axial dimension (H) of the piston washer assembly by moving the proximal part and the distal part relative to each other to increase said axial dimension until the proximal part and the distal part assume a target state where said axial dimension reaches an axial target dimension, and
- mutually interconnecting the first and the second housing components with the piston washer assembly positioned between the piston rod and the piston.

In accordance with the fifth aspect, the step of modifying the axial dimension of the piston washer assembly is carried out while the proximal part and the distal part are situated remotely from at least one of the first and the second sub-assembly. In some embodiments, this is carried out while the proximal part and the distal part of the piston washer assembly are situated remotely from both the first and the second sub-assemblies, i.e. to allow for full operability of the tools required for modifying the axial dimension of the piston washer assembly.

As used herein, the term "insulin" is meant to encompass any drug-containing flowable medicine capable of being passed through a delivery means such as a cannula or hollow needle in a controlled manner, such as a liquid, solution, gel or fine suspension, and which has a blood glucose controlling effect, e.g. human insulin and analogues thereof as well as non-insulins such as GLP-1 and analogues thereof. In the description of exemplary embodiments reference will be made to the use of insulin.

### BRIEF DESCRIPTION OF DRAWINGS

In the following the invention will be further described with reference to the drawings, wherein
fig. 1 shows a perspective view of a prior art pen device,
fig. 2 shows in an exploded view the components of the pen device of fig. 1,
figs. 3A and 3B show in sectional views an expelling mechanism of the pen device of fig. 1 in two states,
figs. 3C - 3E show components of the pen device of fig. 1,
figs. 4 and 5 schematically illustrate the effect of tolerance variations for piston rod position and piston position for four sample pen devices during device assembly,
fig. 6a shows a perspective view of a first embodiment of a piston washer assembly according to the invention,
fig. 6b shows a perspective cross-sectional view of the piston washer assembly shown in fig. 6a,
figs. 6c and 6d are perspective cross-sectional views of a proximal part and a distal part of the piston washer assembly shown in fig. 6a,
fig. 7 schematically illustrates an adjustment procedure for adjusting the piston washer assembly shown in fig. 6a,
fig. 8a and 8b show detailed cross-sectional plan views of the piston washer assembly shown in fig. 6a prior to and after adjustment,
fig. 9a shows a perspective view of a second embodiment of a piston washer assembly according to the invention,
fig. 9b shows a partly cut-away perspective cross-sectional view of the piston washer assembly shown in fig. 9a,
fig. 10 schematically illustrates an adjustment procedure for adjusting the piston washer assembly shown in fig. 9a,
fig. 11a and 11b show detailed cross-sectional plan views of the piston washer assembly shown in fig. 9a prior to and after adjustment, and
fig. 12 shows piston washer assemblies of fig. 9a adjusted for different axial dimension to compensate for tolerance variations in four sample pen devices.

Corresponding reference characters indicate corresponding parts throughout the several views. Although the drawings represent embodiments of the present invention, the drawings are not necessarily to scale, and certain features may be exaggerated or omitted in some of the drawings in order to better illustrate and explain the present invention.

### DESCRIPTION

When in the following terms such as "upper" and "lower", "right" and "left", "horizontal" and "vertical" or similar relative expressions are used, these only refer to the appended figures and not necessarily to an actual situation of use. The shown figures are schematic representations for which reason the configuration of the different structures as well as their relative dimensions are intended to serve illustrative purposes only. When the term member or element is used for a given component it generally indicates that in the described embodiment the component is a unitary component, however, the same member or element may alternatively comprise a number of sub-components just as two or more of the described components could be provided as unitary components, e.g. manufactured as a single injection moulded part. The term "assembly" does not imply that the described components necessarily can be assembled to provide a unitary or functional assembly during a given assembly procedure but is merely used to describe components grouped together as being functionally more closely related.

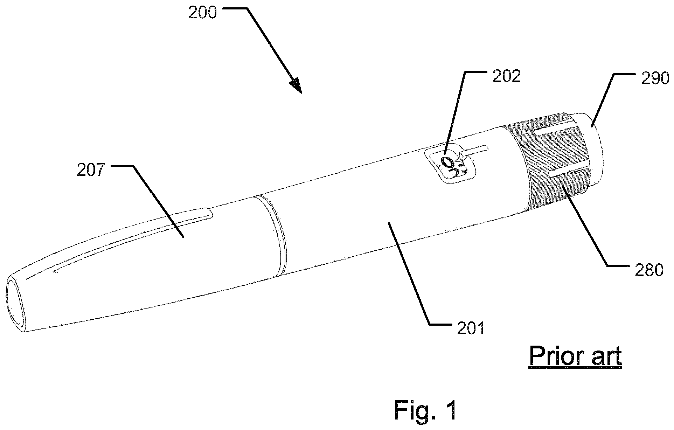

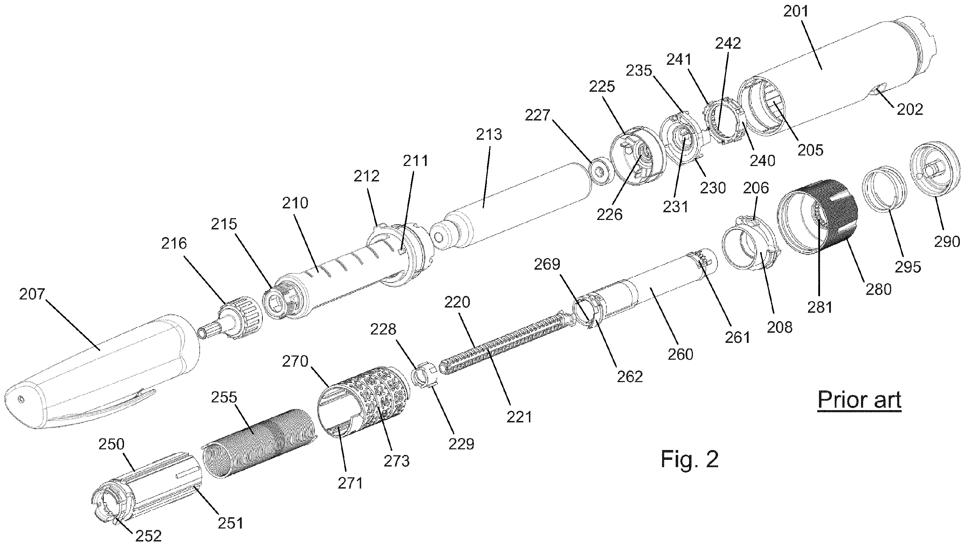

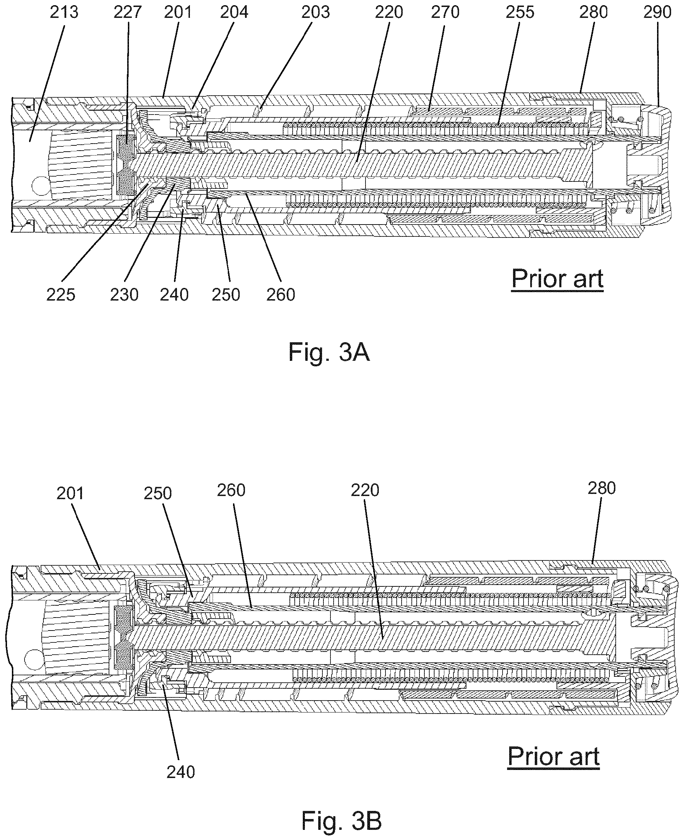

Figs. 1 shows a prior art drug delivery device in the form of a pen-formed auto-injection device 200, i.e. a so-called "injection pen" that includes an expelling mechanism incorporating a spring drive. Fig. 2 shows an exploded view of the prior art auto-injection device 200 shown in fig. 1. Figs. 3A and 3B show cross sectional views of the expelling mechanism of the prior art auto-injection device 200 shown in figs. 1 and 2 where fig. 3A shows the device in dose setting state and fig. 3B shows the device in dose expelling state.

In the present context the device 200 represents a "generic" drug delivery device providing a specific example of a device which, in accordance with the present invention, may be modified in order to obtain a device that provides improved user feedback. As the invention relates to elements of a device which mainly pertains to user feedback, an exemplary embodiment of such a device will be described for better understanding of the invention.

The pen device 200 comprises a cap part 207 and a main part having a proximal body or drive assembly portion with a housing 201 in which a drug expelling mechanism is arranged or integrated, and a distal cartridge holder portion in which a drug-filled transparent cartridge 213 with a distal needle-penetrable septum is arranged and retained in place by a non-removable cartridge holder attached to the proximal portion, the cartridge holder having openings allowing a portion of the cartridge to be inspected as well as distal coupling means 215 allowing a needle assembly to be releasably mounted. The cartridge is provided with a piston driven by a piston rod forming part of the expelling mechanism and may for example contain an insulin, GLP-1 or growth hormone formulation. A proximal-most rotatable dose dial member 280 serves to manually set a desired dose of drug shown in display window 202 and which can then be expelled when the release button 290 is actuated. Depending on the type of expelling mechanism embodied in the drug delivery device, the expelling mechanism may comprise a spring as in the shown embodiment which is strained during dose setting and then released to drive the piston rod when the release button 290 is actuated.

As appears, fig. 1 shows a drug delivery device of the pre-filled type, i.e. it is supplied with a pre-mounted cartridge and is to be discarded when the cartridge has been emptied. In alternative embodiments, and in accordance with the present invention, the drug delivery device may be designed to allow a loaded cartridge to be replaced, e.g. in the form of a "rear-loaded" drug delivery device in which the cartridge holder is adapted to be removed from the device main portion, or alternatively in the form of a "front-loaded" device in which a cartridge is inserted through a distal opening in the cartridge holder which is non-removable attached to the main part of the device.

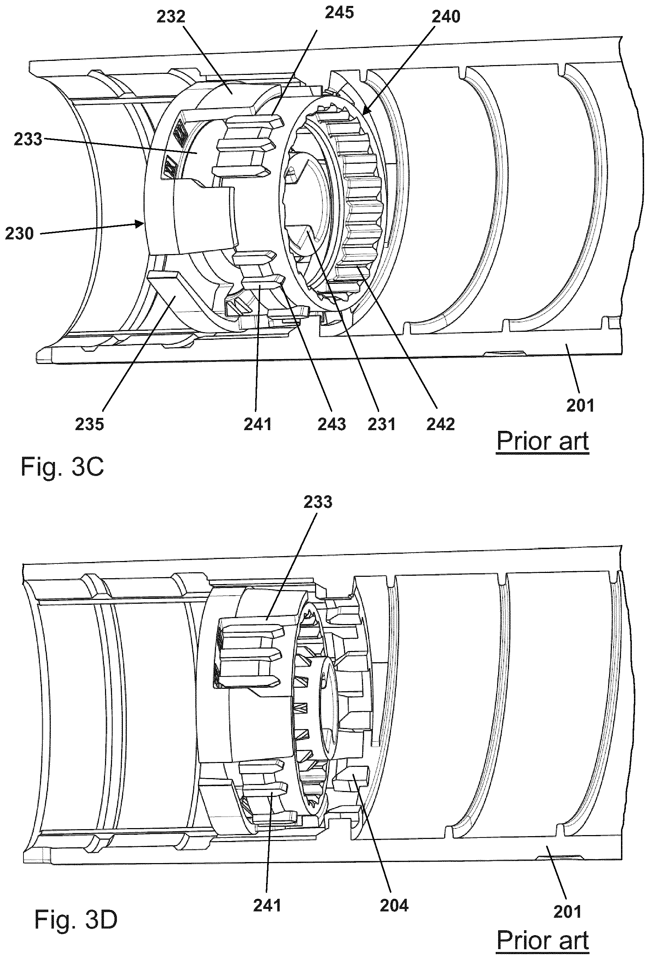

More specifically, referring to fig. 2, the pen comprises a tubular housing 201 with a window opening 202 and onto which a cartridge holder 210 is fixedly mounted, a drug-filled cartridge 213 being arranged in the cartridge holder. The cartridge holder is provided with distal coupling means 215 allowing a needle assembly 216 to be releasably mounted, proximal coupling means in the form of two opposed protrusions 211 allowing a cap 207 to be releasably mounted covering the cartridge holder and a mounted needle assembly, as well as a protrusion 212 preventing the pen from rolling on e.g. a table top. In the housing distal end a nut element 225 is fixedly mounted, the nut element comprising a central threaded bore 226, and in the housing proximal end a spring base member 208 with a central opening is fixedly mounted. A drive system comprises a threaded piston rod 220 having two opposed longitudinal grooves and being received in the nut element threaded bore, a ring-formed piston rod drive element 230 rotationally arranged in the housing, and a ring-formed clutch element 240 which is in rotational engagement with the drive element (see below), the engagement allowing axial movement of the clutch element. The clutch element is provided with outer spline elements 241 adapted to engage corresponding splines on the housing inner surface, this allowing the clutch element to be moved between a rotationally locked proximal position, in which the splines are in engagement, and a rotationally free distal position in which the splines are out of engagement. As just mentioned, in both positions the clutch element 240 is rotationally locked to the drive element 230. The drive element comprises a central bore with two opposed protrusions 231 in engagement with the grooves on the piston rod whereby rotation of the drive element results in rotation and thereby distal axial movement of the piston rod due to the threaded engagement between the piston rod and the nut element. The drive element further comprises a pair of opposed circumferentially extending flexible ratchet arms 235 adapted to engage corresponding ratchet teeth 205 arranged on the housing inner surface. The drive element and the clutch element comprise cooperating coupling structures rotationally locking them together but allowing the clutch element to be moved axially, this allowing the clutch element to be moved axially to its distal position in which it is allowed to rotate, thereby transmitting rotational movement from the dial system (see below) to the drive system. The interaction between the clutch element, the drive element and the housing will be shown and described in greater detail with reference to figs. 3C and 3D.

On the piston rod an end-of-content (EOC) member 228 (EOC limiter) is threadedly mounted and on the distal end a washer 227 is rotationally mounted. The EOC member comprises a pair of opposed radial projections 229 for engagement with the reset tube (see below).

The dial system comprises a ratchet tube 250, a reset tube 260, a scale drum 270 with an outer helically arranged row of dose numerals, a user-operated dose dial member 280 for setting a dose of drug to be expelled, a release button 290 and a torque spring 255 (see fig. 3A and 3B). The reset tube is mounted axially locked inside the ratchet tube but is allowed to rotate a few degrees (see below). The reset tube comprises on its inner surface two opposed longitudinal grooves 269 adapted to engage the radial projections 229 of the EOC member, whereby the EOC can be rotated by the reset tube but is allowed to move axially. The clutch element is mounted axially locked on the outer distal end portion of the ratchet tube 250, this providing that the ratchet tube can be moved axially in and out of rotational engagement with the housing via the clutch element. The dose dial member 280 is mounted axially locked but rotationally free on the housing proximal end, the dose dial member being under normal operation rotationally locked to the reset tube (see below), whereby rotation of dose dial member results in a corresponding rotation of the reset tube and thereby the ratchet tube. The release button 290 is axially locked to the reset tube but is free to rotate. A return spring 295 provides a proximally directed force on the button and the thereto mounted reset tube. The scale drum 270 is arranged in the circumferential space between the ratchet tube and the housing, the drum being rotationally locked to the ratchet tube via cooperating longitudinal splines 251, 271 and being in rotational threaded engagement with the inner surface of the housing via cooperating thread structures 203, 273, whereby the row of numerals passes the window opening 202 in the housing when the drum is rotated relative to the housing by the ratchet tube. The torque spring is arranged in the circumferential space between the ratchet tube and the reset tube and is at its proximal end secured to the spring base member 208 and at its distal end to the ratchet tube, whereby the spring is strained when the ratchet tube is rotated relative to the housing by rotation of the dial member. A ratchet mechanism with a flexible ratchet arm 252 is provided between the ratchet tube and the clutch element, the latter being provided with an inner circumferential teeth structures 242, each tooth providing a ratchet stop such that the ratchet tube is held in the position to which it is rotated by a user via the reset tube when a dose is set. In order to allow a set dose to be reduced a ratchet release mechanism 262 is provided on the reset tube and acting on the ratchet tube, this allowing a set dose to be reduced by one or more ratchet increments by turning the dial member in the opposite direction, the release mechanism being actuated when the reset tube is rotated the above-described few degrees relative to the ratchet tube.

Having described the different components of the expelling mechanism and their functional relationship, operation of the mechanism will be described next with reference mainly to figs. 3A and 3B.

The pen mechanism can be considered as two interacting systems, a dose system and a dial system, this as described above. During dose setting the dial mechanism rotates and a torsion spring of the spring drive is loaded. The dose mechanism is locked to the housing and cannot move. When the push button is pushed down, the dose mechanism is released from the housing and due to the engagement to the dial system, the torsion spring will now rotate back the dial system to the starting point and rotate the dose system along with it.

The central part of the dose mechanism is the piston rod 220, the actual displacement of the piston being performed by the piston rod. During dose delivery, the piston rod is rotated by the drive element 230 and due to the threaded interaction with the nut element 225 which is fixed to the housing, the piston rod moves forward in the distal direction. Between the rubber piston and the piston rod, the piston washer 227 is placed which serves as an axial bearing for the rotating piston rod and evens out the pressure on the rubber piston. As the piston rod has a non-circular cross section where the piston rod drive element engages with the piston rod, the drive element is locked rotationally to the piston rod, but free to move along the piston rod axis. Consequently, rotation of the drive element results in a linear forwards movement of the piston. The drive element is provided with small ratchet arms 234 which prevent the drive element from rotating clockwise (seen from the push button end). Due to the engagement with the drive element, the piston rod can thus only move forwards. During dose delivery, the drive element rotates anti-clockwise and the ratchet arms 235 provide the user with small clicks due to the engagement with the ratchet teeth 205, e.g. one click per unit of insulin expelled.

Turning to the dial system, the dose is set and reset by turning the dose dial member 280. When turning the dial, the reset tube 260, the EOC member 228, the ratchet tube 250 and the scale drum 270 all turn with it. As the ratchet tube is connected to the distal end of the torque spring 255, the spring is loaded. During dose setting, the arm 252 of the ratchet performs a dial click for each unit dialed due to the interaction with the inner teeth structure 242 of the clutch element. In the shown embodiment the clutch element is provided with 24 ratchet stops providing 24 clicks (increments) for a full 360 degrees rotation relative to the housing. The spring is preloaded during assembly which enables the mechanism to deliver both small and large doses within an acceptable speed interval. As the scale drum is rotationally engaged with the ratchet tube, but movable in the axial direction and the scale drum is in threaded engagement with the housing, the scale drum will move in a helical pattern when the dial system is turned, the number corresponding to the set dose being shown in the housing window 202.

The ratchet 252, 242 between the ratchet tube and the clutch element 240 prevents the spring from turning back the parts. During resetting, the reset tube moves the ratchet arm 252, thereby releasing the ratchet click by click, one click corresponding to one unit IU of insulin in the described embodiment. More specifically, when the dial member is turned clockwise, the reset tube simply rotates the ratchet tube allowing the arm of the ratchet to freely interact with the teeth structures 242 in the clutch element. When the dial member is turned counter-clockwise, the reset tube interacts directly with the ratchet click arm forcing the click arm towards the centre of the pen away from the teeth in the clutch, thus allowing the click arm on the ratchet to move "one click" backwards due to torque caused by the loaded spring.

To deliver a set dose, the release button 290 is pushed in the distal direction by the user as shown in fig. 3B. The reset tube 260 decouples from the dial member and subsequently the clutch element 240 disengages the housing splines 204. Now the dial mechanism returns to "zero" together with the drive element 230, this leading to a dose of drug being expelled. It is possible to stop and start a dose at any time by releasing or pushing the push button at any time during drug delivery. A dose of less than 5 IU normally cannot be paused, since the rubber piston is compressed very quickly leading to a compression of the rubber piston and subsequently delivery of insulin when the piston returns to the original dimensions.

The EOC feature prevents the user from setting a larger dose than left in the cartridge. The EOC member 228 is rotationally locked to the reset tube, which makes the EOC member rotate during dose setting, resetting and dose delivery, during which it can be moved axially back and forth following the thread of the piston rod. When it reaches the proximal end of the piston rod a stop is provided, this preventing all the connected parts, including the dial member, from being rotated further in the dose setting direction, i.e. the now set dose corresponds to the remaining drug content in the cartridge.

The scale drum 270 is provided with a distal stop surface adapted to engage a corresponding stop surface on the housing inner surface, this providing a maximum dose stop for the scale drum preventing all the connected parts, including the dial member, from being rotated further in the dose setting direction. In the shown embodiment the maximum dose is set to 80 IU. Correspondingly, the scale drum is provided with a proximal stop surface adapted to engage a corresponding stop surface on the spring base member, this preventing all the connected parts, including the dial member, from being rotated further in the dose expelling direction, thereby providing a "zero" stop for the entire expelling mechanism. In the following, the position that the dial member assumes after completion of the expelling of a set dose will be referred to as the "zero dose position".

To prevent accidental over-dosage in case something should fail in the dialing mechanism allowing the scale drum to move beyond its zero-position, the EOC member serves to provide a security system. More specifically, in an initial state with a full cartridge the EOC member is positioned in a distal-most axial position in contact with the drive element. After a given dose has been expelled the EOC member will again be positioned in contact with the drive element. Correspondingly, the EOC member will lock against the drive element in case the mechanism tries to deliver a dose beyond the zero-position. Due to tolerances and flexibility of the different parts of the mechanism the EOC will travel a short distance allowing a small "over dose" of drug to be expelled, e.g. 3-5 IU of insulin.

The expelling mechanism further comprises an end-of-dose (EOD) click feature providing a distinct feedback at the end of an expelled dose informing the user that the full amount of drug has been expelled. More specifically, the EOD function is made by the interaction between the spring base and the scale drum. When the scale drum returns to zero, a small click arm 206 on the spring base is forced backwards by the progressing scale drum. Just before "zero" the arm is released and the arm hits a countersunk surface on the scale drum.

The shown mechanism is further provided with a torque limiter in order to protect the mechanism from overload applied by the user via the dose dial member. This feature is provided by the interface between the dose dial member and the reset tube which as described above are rotationally locked to each other. More specifically, the dose dial member is provided with a circumferential inner teeth structure 281 engaging a number of corresponding teeth arranged on a flexible carrier portion 261 of the reset tube. The reset tube teeth are designed to transmit a torque of a given specified maximum size, e.g. 150-300 Nmm, above which the flexible carrier portion and the teeth will bend inwards and make the dose dial member turn without rotating the rest of the dial mechanism. Thus, the mechanism inside the pen cannot be stressed at a higher load than the torque limiter transmits through the teeth.

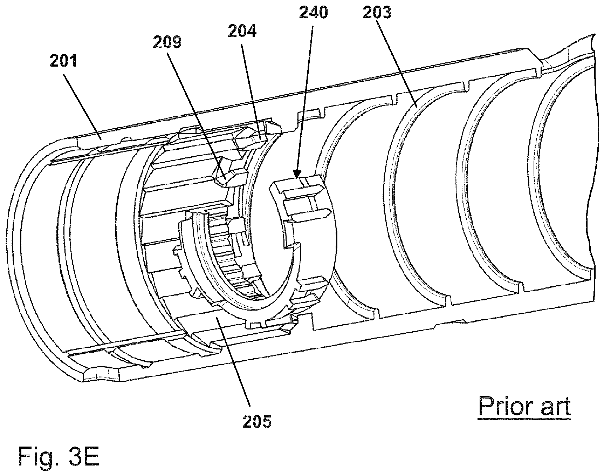

In fig. 3C the clutch element, the drive element and the housing (in partial) are shown in the dose setting state, and in fig. 3D the same components are shown in the expelling state. As appears, the piston rod on which the drive element is arranged and the ratchet tube on which the clutch element is mounted are not shown. To better show the structures provided on the inner surface of the housing fig. 3E shows a partial clutch element 240 arranged in the housing 201.

The inner surface of the housing 201 comprises a circumferential ring-formed array of axially oriented spline elements 204 protruding into the interior, each having a pointed distal end 209, as well as a circumferential ring-formed array of one-way ratchet teeth 205. The inner surface further comprises a male helical thread 203 adapted to engage the female helical thread 273 on the scale drum 270. A distal circumferential groove is formed to engage and mount the nut element 225. The clutch element 240 comprises an inner circumferential ring-formed array of ratchet teeth 242 adapted to engage the ratchet arm 252 on the ratchet tube 250, and an outer circumferential ring-formed array of axially oriented spline elements 241 adapted to engage the spline elements 204 of the housing as well as the coupling slots in the drive element (see below), each spline having a pointed proximal end 243. The drive element 230 comprises a pair of opposed coupling portions each comprising two proximally extending skirt portions 232 between which an axially extending coupling slot 233 is formed, the slot being adapted to engage a portion of the clutch element spline elements. In this way the engaging surfaces serve to transmit a rotational force and thereby torque from the clutch element to the drive element in the expelling state. The drive element further comprises a pair of opposed circumferentially extending flexible ratchet arms adapted to engage the ring-formed array of one-way ratchet teeth 205. During dose delivery, the drive element rotates anti-clockwise and the ratchet arms 235 also provide the user with small clicks due to the engagement with the ratchet teeth 205, e.g. one click per unit of insulin expelled. In the shown embodiment 24 ratchet teeth are provided corresponding to 15 degrees rotation per unit of insulin. The central bore of the drive element comprises two opposed protrusions 231 adapted to engage with the axially oriented grooves on the piston rod.

In the dose setting state shown in fig. 3C the spline elements 241 of the clutch element are in engagement with the spline elements 204 of the housing thereby rotationally locking the clutch element relative to the housing. As can be seen from fig. 3C a group of clutch spline elements are received in the corresponding coupling slot with a slight rotational play. In the expelling state shown in fig. 3D the spline elements 241 of the clutch element are moved distally out of engagement with the spline elements 204 of the housing thereby allowing rotation of the clutch element relative to the housing. As can be seen from fig. 3D the group of clutch spline elements are now received in the corresponding coupling slot without rotational play.

Fig. 3C shows the clutch element 240 showing the above-described inner circumferential ring-formed array of ratchet teeth 242 and the outer circumferential ring-formed array of axially oriented spline elements 241. As appears, the spline elements are not arranged equidistantly on the ring but in groups, the groups comprising two opposed coupling groups 245 serving as the coupling means engaging the coupling slots 233. Whereas thus only some of the spline elements serve as coupling means between the clutch element and the drive element they all serve as coupling means between the clutch element and the housing splines 204.

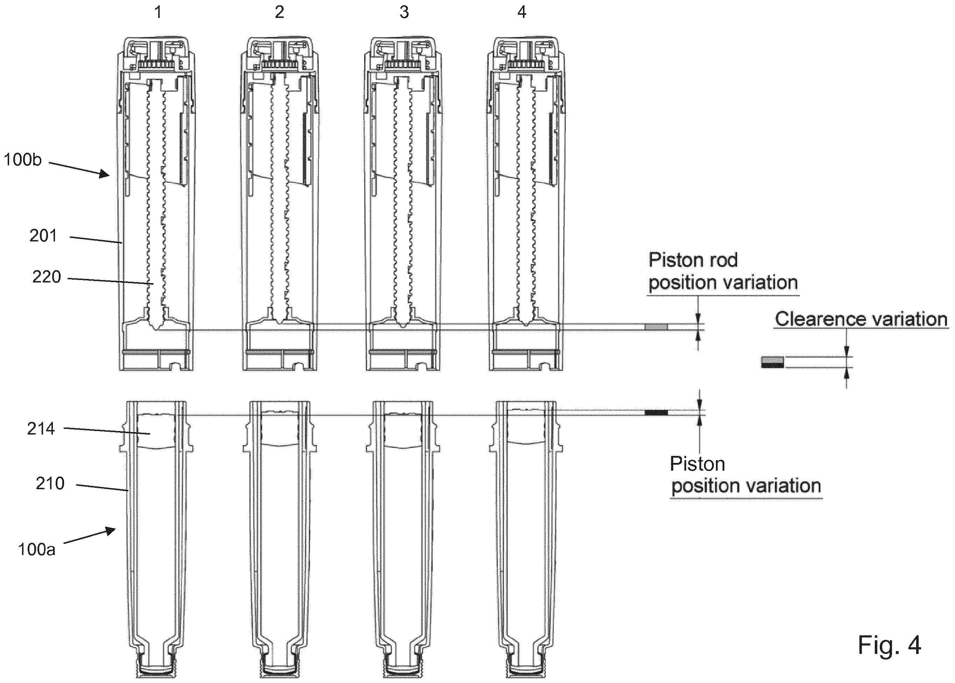

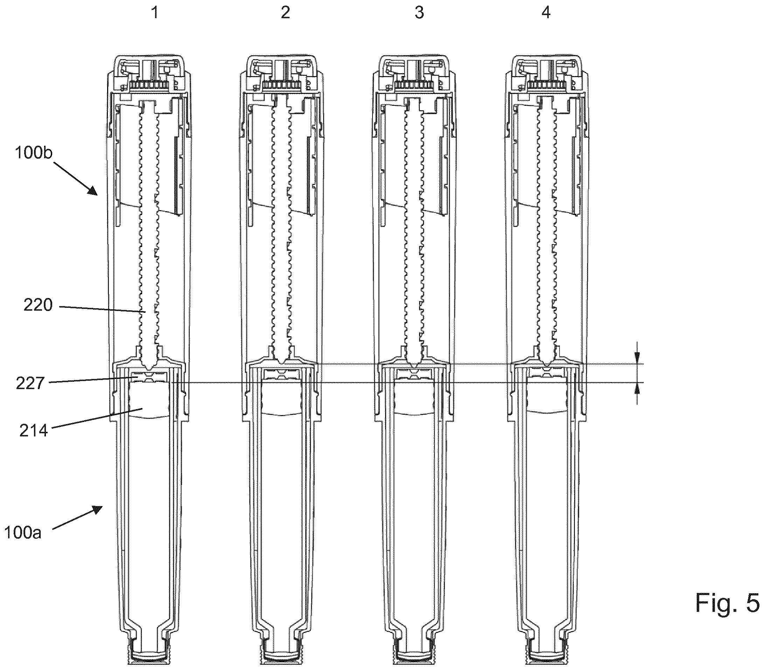

Production tolerances on the piston rod, the expelling mechanism, cartridge body, cartridge filling level and other components result in variations in piston rod position and piston position in each device during assembly. Such variation is schematically represented in figs. 4 and 5 for four different pen samples. In these figures, for reasons of clarity, only components serving understanding the problem associated with tolerance variations are shown whereas other components have been omitted from the drawings.

In exemplary embodiments, during assembly of the pen device, the cartridge may be initially inserted in the cartridge holder to form a first sub-assembly 100a. The dose setting and expelling mechanism including the piston rod is mounted relative to the housing to form a second sub-assembly 100b. The position of the piston 214 of the cartridge varies from one cartridge to another. To be able to compensate for tolerance variation, the position of the cartridge is therefore determined relative to a fixed reference point on the cartridge holder 210. Also the piston rod position in the housing can be determined relative to a fixed reference point on the housing 201.

Before the two sub-assemblies 100a and 100b are finally attachably coupled to each other a fixed dimension piston washer 227 is brought into engagement with the piston 214 of the cartridge. If no further compensation of the tolerance variations were made, the final pen assemblies would assume the states shown in fig. 5 where potentially axial clearance between the piston rod and the piston washer and/or the piston washer and the piston would be present wherein the magnitude of the axial clearance would vary from different samples.

Hence, during assembly, and in order to minimize a potential axial clearance between the piston rod and the piston of the cartridge, positioning may be carried out by initially positioning the piston rod 220 in a nominal position. Due to tolerances various different clearance gaps between the piston and the piston rod will show when the distal and proximal subassemblies of each sample are permanently secured together. On the basis of measurements or estimations, which may be performed at different steps of the assembly process, the actual gap in each sample may be eliminated or at least partly reduced by operating the dose setting and expelling mechanism. Automated operation of the dose setting and expelling mechanism may be carried out either after final assembly or prior to final coupling of the different subassemblies. However such compensation procedure means that the end-of-content mechanism will be operated to a lesser or higher degree even before the device is shipped to the user meaning that the experienced total doseable volume varies from sample to sample. Generally such variations and inconsistencies from one sample to another should be avoided as this may provide the impression that the quality of the device could be somewhat non-optimal.

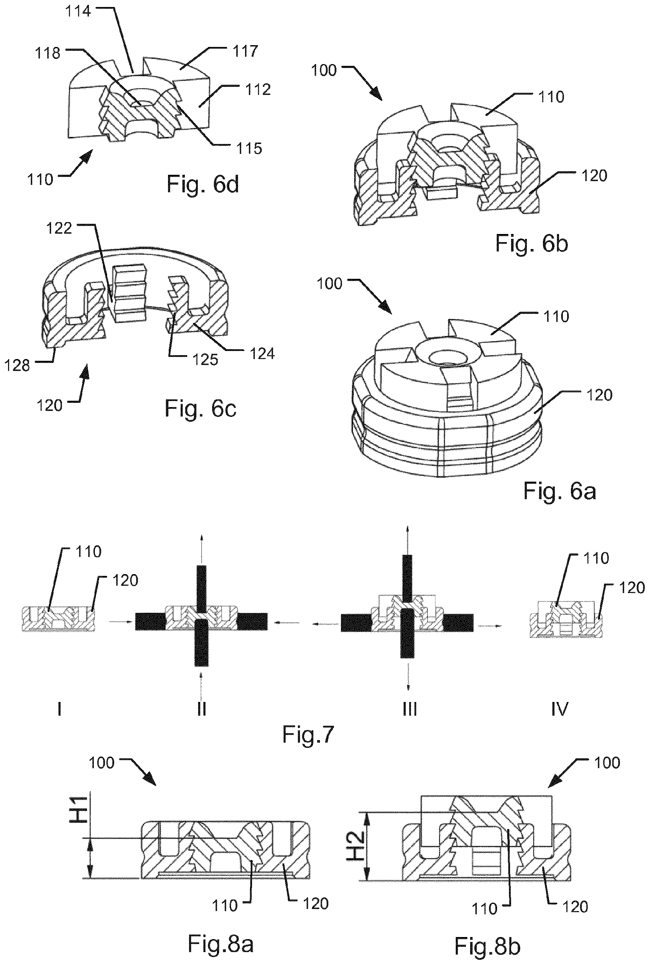

Turning to fig. 6a through fig. 8, in accordance with an aspect of the invention, a first embodiment of a variable size piston washer assembly 100 will be described. In an injection device, such as the prior art device described above, the washer assembly 100 is intended for replacing the washer 227 seeking to eliminate or reduce the above problem of varying total doseable volume across different pen samples. However, this is only a non-limiting example and the piston washer assembly as herein described may be used in conjunction with other injection devices, whether single shot or multi-shot injection devices.

The piston washer assembly 100 serves in a drug delivery device to transfer a distally directed axial force from a piston rod towards a piston of a held cartridge. The piston washer assembly 100 comprises a proximal part 110 adapted for engagement with the distal portion of the piston rod and a distal part 120 configured to engage and abut the piston of a held cartridge. The shape of the distal part 120 is generally complementary to the shape of the proximal part 110. One or both of the distal part 120 and the proximal part 110 is made of a sturdy yet resilient material where the resiliency serves for enabling relative movement of the distal part and the proximal part during an adjustment procedure for varying the axial size of the piston washer. The piston washer assembly 100 is in fig. 6a and 6b shown in a state where an axial size of the piston washer assembly has already been adjusted during an adjustment process.

As shown in fig. 6c the distal part 120 comprises a peripheral portion exhibiting a distal facing surface 128 along the perimeter of the distal part. The distal facing surface 128 is adapted to lie flush against the piston of a held cartridge. In the shown embodiment four rib-formed sections 124, angled 90 degrees apart, extend radially inwards from the peripheral portion. Each rib section 124 carries a stepped surface facing radially inwards and thus defining a plurality of inwardly directed resilient teeth 125. Axially extending surfaces 122 limits the peripheral width of the stepped surface of each rib section 124.

Fig. 6d show the proximal part 110 which is of generally cylindrical shape where the radially outer surface has four axial tracks 114 formed therein, the axial tracks 114 being spaced apart by 90 degrees. Each of the rib-formed sections 124 of the distal part 120 fits snuggly into a respective axial track 114 of the proximal part so that the distal part 120 and the proximal part 110 can be arranged coaxially and axially overlapping. The proximal surface 117 of the proximal part 110 may comprise a recessed central area 118 that act as a bearing surface for engagement with the distal end of the piston rod allowing low-friction rotational movement of the piston rod relative to the proximal part 110 of the piston washer assembly 100. In the shown embodiment, the central area 118 includes an annular surface which may be formed exhibiting a conical or part-spherical surface. The central area 118 is shown as a recessed area configured to engage a convex annular end surface of the piston rod but may alternatively be formed as an area that protrudes from the proximal surface 117 to engage with a concave annular end surface of the piston rod.

When the distal part 120 and the proximal part 110 is axially overlapping, the axially extending surfaces 122 of distal part 120 mate and lie flush against axial extending surfaces 112 of the proximal part. Thus the interfaces between the distal part 120 and the proximal part 110 form a linear guide allowing axial movement there between. However, due to the teeth 125 of the distal part 120 which cooperate with radially outwards facing resilient teeth 115 of the proximal part 110 only one-way movement is possible, e.g. the proximal part 110 may be moved relative to the distal part 120 in the proximal direction only. Hence, a one-way linear ratchet is provided between proximal part 110 and distal part 120 providing an incremental detent mechanism.

Each of the proximal part and the distal part is preferably made by an injection process. In certain embodiments, the two parts can be made by a two-shot injection molding process wherein the first part is made in a first injection molding shot and the second part is made in a second subsequent injection molding shot which partly utilizes the surfaces of the first part as boundaries for forming the molded second part. Suitable materials may be selected Polypropylene (PP) for one of the parts and Polyoxymethylene plastic (POM) for the other part as these materials do not stick well to each other and will allow the two parts to be subsequently moved axially relative to each other.

If the proximal part and the distal part are made by separate processes, the proximal part 110 may be inserted from the distal end of the distal part 120 and moved into engagement so that a first pair of corresponding teeth 115/125 engage.

Non-limiting examples of an incremental detent mechanism allowing the axial dimension to be increased in incremental steps may be designed for axial movements with axial incremental steps of unit size ΔH which may be selected in the order of 0.1 mm to 0.4 mm. An example of a proper number of unique relative axial positions between the proximal part 110 and the distal part 120 may be selected as three to five unique positions. However, in embodiments with finer unit-step resolution, the number of unique positions may be even higher, such as 6 to 12 unique positions.

Fig. 7 schematically illustrates an adjustment procedure for adjusting the piston washer assembly 100. In the initial state shown in fig. 7(I) the piston washer assembly 100 exhibits an axial dimension H of initial size H1 (cf. fig. 8a). Utilizing an automated tool for the adjustment procedure, a first tool part is designed to grip the distal part 120. A second tool part pushes the proximal part 110 in the proximal direction, i.e. from the distal side of the piston washer assembly. As shown in fig. 7(II), to obtain increased control, a further third tool part may abut the proximal face of the proximal part 110 and move in unison with the second tool part. By moving the second and third tool parts relative to the first tool part the proximal part and the distal part is caused to assume a target state where said axial dimension enters into an axial target dimension as shown in fig. 7(III). The tool parts may thereafter be removed from the assembly. As shown in fig. 7 (IV) and fig. 8b, the final adjusted piston washer assembly 100 having the desired target dimension H2 is formed.

Due to the one-way detent mechanism 115/125 the adjusted piston washer assembly 100 is able to withstand axial compression without collapsing in the axial dimension.

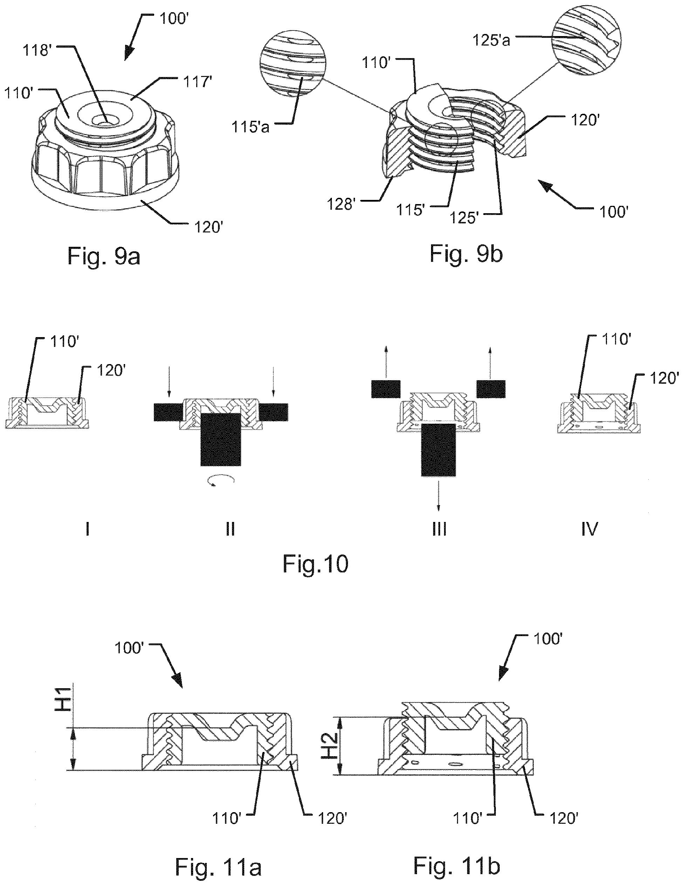

Fig. 9a through fig. 11b show a second embodiment of a variable size piston washer assembly 100'. The piston washer assembly 100' comprises a proximal part 110' adapted for engagement with the distal portion of the piston rod and a distal part 120' configured to engage and abut the piston of a held cartridge. The shape of the distal part 120' is generally cylindrical. Also the proximal part 110' is generally cylindrical and adapted to be arranged coaxilly with the distal part 120 with the distal part surrounding the proximal part. Again, one or both of the distal part 120' and the proximal part 110' may be made of a resilient material. The piston washer assembly 100' is in fig. 9a and 9b shown in a state where an axial size of the piston washer assembly has already been adjusted during an adjustment process.

As shown in fig. 9b the distal part 120' defines an axial bore provided with a thread configuration 125'. An outer cylindrical surface of the proximal part 110' exhibits a corresponding thread configuration 115' made for engaging with thread configuration 125'. Relative rotational movement in a first rotational direction between the proximal part 110' and the distal part 120' increases the axial dimension of the piston washer assembly 100'.

As shown in fig. 9a, in order to assist automated adjustment of the piston washer assembly 100', the external surface of distal part 120' is provided with geometries enabling a tool to engage with a sufficient torque transferring grip. Similarly, although not visible on the drawings, an internal opening at the distal side of the proximal part 110' is provided with similar geometries allowing a suitable shaped tool, such as a hexagon tool, to be inserted in the opening and exerting torque on the proximal part 110'.

In the shown embodiment, an incremental positioning mechanism is provided between the distal part and the proximal part. This is provided as cooperating rotational detent geometries 115'a and 125'a arranged at different locations in the thread configurations 115' and 125' (see enlarged sections of fig. 9b). When the piston washer assembly 100' is positioned in a pen device, the incremental positioning mechanism prevent the inherent tendency to cause relative rotational movement between the proximal part 110' and the distal part 120' when excessive axial forces act to compress the piston washer assembly 100'. However, relative rotational movement is possible during the adjustment procedure as sufficient torque may be applied by tools during adjustment of the piston washer assembly 100'.

In other embodiments the cooperating rotational detent geometries 115'a and 125'a may be omitted. Instead, the rotational fixation may be obtained by friction alone.

By incorporating the threaded connection between the distal part 120' and the proximal part 110', an increase in resolution of the adjustment may be obtained relative to the first embodiment. However, also for the first embodiment of the piston washer assembly 100, if so desired, the resolution may be increased if several individual sets of cooperating teeth 115/125 are provided and if these have been arranged slightly axially offset relative to other sets of cooperating teeth 115/125.

Fig. 10 schematically illustrates an adjustment procedure for adjusting the piston washer assembly 100' according to the second embodiment. In the initial state shown in fig. 10(I) the piston washer assembly 100' exhibits an initial axial dimension H1 (cf. fig. 11a). Utilizing an automated tool for the adjustment procedure, a first tool part is designed to grip the distal part 120'. A second tool part is inserted axially from the distal side of the piston washer assembly 100' and made to engage the internal opening at the distal side of the proximal part 110', see fig. 10(II). By rotating the first tool part and second tool part relative to each other the proximal part and the distal part is caused to assume a target state where said axial dimension enters into an axial target dimension as shown in fig. 10(III). The tool parts may thereafter be removed from the assembly. As shown in fig. 10 (IV) and fig. 11b, the final adjusted piston washer assembly 100' having the desired target dimension H2 is formed.

The adjusted piston washer assembly 100 is able to withstand axial compression without collapsing in the axial dimension, this partly due to the rotational detent geometries 115'a/125'a.

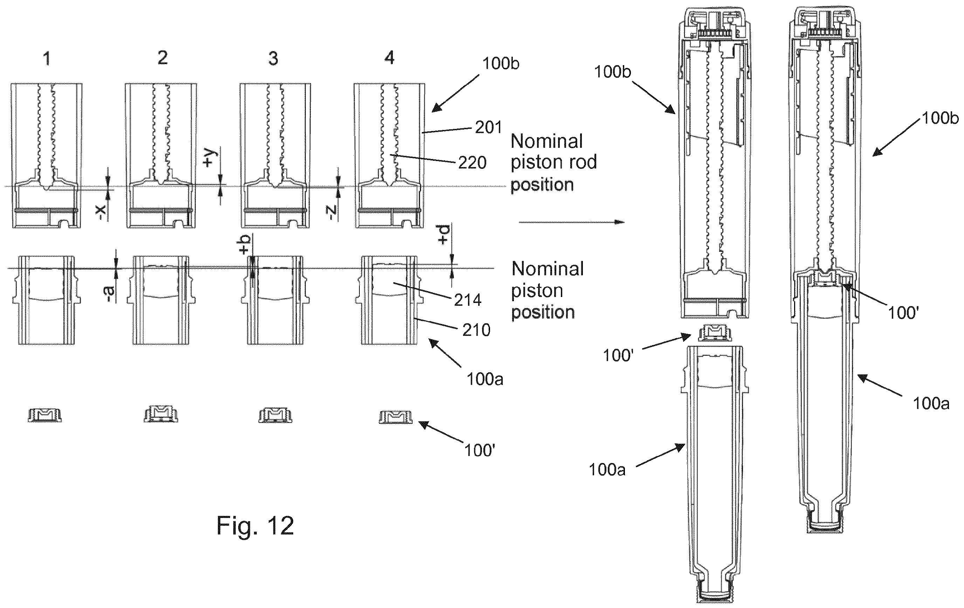

Fig. 12 schematically shows key components for four different pen samples, where each pen sample exhibit tolerance variations both with respect to the nominal piston position in the first sub-assembly and with respect to the nominal piston rod position of the second sub-assembly. For each first sub-assembly and second sub-assembly designated for subsequently mating and coupling, in accordance with determined or measured tolerance variations for the sub-assemblies, a designated piston washer assembly is adjusted to eliminate, or at least partly compensate, for the axial clearance between the piston rod and the piston.

As shown in the right hand side of fig. 12, the piston washer assembly is subsequently arranged between the piston rod and the piston thereby partly or completely filling the axial clearance between these components. As such, operation of the dose setting and expelling mechanism is not required during assembly operations of the pen devices. However, if desired, for example for testing the dose setting and expelling mechanisms, a dose setting and expelling operation of a standardized magnitude may be provided. This may be compensated for when preparing and adjusting the individual piston washer assemblies. In accordance herewith, the total dosage volume for each pen device can be made constant across individual pen samples.

While certain features of the invention have been illustrated and described herein, many modifications, substitutions, changes, and equivalents will now occur to those of ordinary skill in the art. It is, therefore, to be understood that the appended claims are intended to cover all such modifications and changes as fall within the true spirit of the invention.

## Claims

1. A piston washer assembly for use in a drug delivery device for transferring a distally directed axial force from a piston rod towards a piston of a held cartridge, the piston washer assembly (100, 100') comprising:
- a proximal part (110, 110') adapted for engagement with the distal portion of a piston rod (220), the proximal part (110, 110') defining a first set of cooperating geometries (115,115'), and
- a distal part (120, 120') configured to engage and abut a piston (214) of a held cartridge (213) and to engage the proximal part (110, 110'), wherein the distal part (120, 120') defines a second set of cooperating geometries (125, 125') engaging the first set of cooperating geometries (115,115'),
wherein, in an initial state of the assembly, the proximal part (110, 110') and the distal part (120, 120') are positioned relatively to each other to define an initial axial dimension (H, H1) for the assembly, and wherein the proximal part (110, 110') and the distal part (120, 120') are movable relative to each other by moving the first set of cooperating geometries (115,115') and the second set of cooperating geometries (125, 125') relative to each other to increase said axial dimension (H) until the proximal part (110, 110') and the distal part (120, 120') assume a target state where said axial dimension (H) reaches an axial target dimension (H2), and
wherein the first set of cooperating geometries (115,115') and the second set of cooperating geometries (125, 125') are configured to prevent the distal part (120, 120') and the proximal part (110, 110') from being moved relative to each other when forces act on the proximal part (110, 110') and the distal part (120, 120') to decrease said axial target dimension (H2).

2. A piston washer assembly as defined in claim 1, wherein the first set of cooperating geometries (115,115') and the second set of cooperating geometries (125, 125') are configured to provide a detent mechanism (115a, 125a) allowing the axial dimension (H) to be increased while preventing the axial dimension (H) to be decreased.

3. A piston washer assembly as defined in any of the claims 1-2, wherein the first set of cooperating geometries (115,115') and the second set of cooperating geometries (125, 125') define a one-way incremental detent mechanism allowing the axial dimension (H) to be increased in incremental steps of unit size ΔH, and wherein, when the proximal part (110, 110') and the distal part (120, 120') assume the target state, the first set of cooperating geometries (115,115') and the second set of cooperating geometries (125, 125') prevent movement of magnitude ΔH of the first part and the second part relative to each other acting to reduce the axial dimension (H).

4. A piston washer assembly as defined in any of the claims 1-3, wherein the first set of cooperating geometries (115') defines a first thread configuration and the second set of cooperating geometries (125') defines a second thread configuration engaging the first thread configuration, and wherein relative rotational movement in a first rotational direction between the proximal part (110') and the distal part (120') increases the axial dimension (H).

5. A piston washer assembly as defined in claim 4, wherein an incremental positioning mechanism is arranged between the proximal part (110') and the distal part (120') causing said relative rotational movement between the proximal part (110, 110') and the distal part (120') to occur in incremental angular steps.

6. A piston washer assembly as defined in claim 5, wherein said incremental positioning mechanism is provided as cooperating detent geometries arranged in the first thread configuration (115') and the second thread configuration (125').

7. A piston washer assembly as defined in any of the claims 1-3, wherein the first set of cooperating geometries (115) and the second set of cooperating geometries (125) define a linear guide allowing linear axial movement between the proximal part (110) and the distal part (120) and wherein the first set of cooperating geometries (115) and the second set of cooperating geometries (125) define a toothed one-way ratchet engagement allowing the axial dimension (H) to be increased but preventing the axial dimension (H) to be decreased.

8. A piston washer assembly as defined in any of the claims 1-7, wherein the piston washer assembly (100,100') defines a peripheral section having a diameter of a first magnitude and wherein the maximum settable axial dimension (H) is smaller than the first magnitude.

9. A drug delivery device for expelling a dose of drug from a held cartridge, the drug delivery device defining a distal drug outlet end and an opposite proximal end and comprising:
- a housing component (210) holding a cartridge (213) comprising a liquid drug and a piston (214) slideable arranged therein in an axial direction,
- a dose setting and expelling mechanism coupled to the housing component (210) and comprising a piston rod (220) for exerting a force on the piston (214) of the cartridge (213) in a distal direction for expelling a dose, and
wherein a piston washer assembly (100, 100') according to any of the claims 1-8 is arranged axially between the piston (214) of the cartridge (213) and the piston rod (220).

10. A drug delivery device as defined in claim 9, wherein the piston rod (220) rotates during dose expelling and wherein the piston rod (220) is rotatably arranged relative to the piston washer assembly (100,100') to allow the piston rod (220) to rotate while the piston washer assembly (100,100') remains non-rotatable.

11. A method of preparing a piston washer assembly (100,100') as defined in any of the claims 1-10 for use in a drug delivery device for transferring a distally directed axial force from a piston rod towards a piston of a held cartridge, comprising the steps of:
- providing the proximal part (110, 110') and the distal part (120, 120') in an initially engaged state, wherein the proximal part (110, 110') and the distal part (120, 120') are positioned relatively to each other to define an initial axial dimension (H,H1) for the piston washer assembly (100, 100'),
- estimating a desired axial target dimension (H2) for the piston washer assembly (100, 100'), and
- moving the proximal part (110, 110') and the distal part (120, 120') relative to each other to increase said axial dimension (H) until the proximal part (110, 110') and the distal part (120, 120') assume a target state where said axial dimension (H) reaches the desired axial target dimension (H2).

12. A method of preparing a piston washer assembly as defined in claim 11, comprising the steps of:
- providing one of the distal part (120, 120') and the proximal part (110, 110') as a first injection shot in a multi-shot injection molding process, and
- providing the other of the distal part (120, 120') and the proximal part (110, 110') as a further injection shot in a multi-shot injection molding process so that the first set of cooperating geometries (115,115') and the second set of cooperating geometries (125, 125') at least partly engage.

13. A method of preparing a piston washer assembly as defined in any of the claims 11-12, wherein the step of moving the proximal part (110, 110') and the distal part (120, 120') relative to each other to increase said axial dimension (H) until the proximal part (110, 110') and the distal part assume a target state where said axial dimension (H) reaches the desired axial target dimension (H2) comprises the steps of:
- maintaining the distal part (120, 120') engaged with a first tool part,
- maintaining the proximal part (110, 110') engaged with a second tool part,
- moving the first tool part and the second tool part relative to each other while measuring, determining or estimating the size of said axial dimension (H) of the piston washer assembly (100, 100').

14. A method of assembling a drug delivery device as defined in any of the claims 9-10, comprising the steps of:
- positioning the cartridge (213) comprising a liquid drug and a piston (214) slideably arranged therein in a first housing component (210) to form a first sub-assembly (100a),
- positioning the dose setting and expelling mechanism comprising the piston rod (220) in a second housing component (201) to form a second sub-assembly (100b),
- determining the axial position of the piston (214) with respect to the first housing component (210),
- determining the axial position of the piston rod (220) with respect to the second housing component (201),
- determining or estimating the size of axial clearance between the piston rod (220) and the piston (213) if the first sub-assembly (100a) and the second sub-assembly (100b) were assembled and selecting a target axial dimension (H2) for a piston washer assembly (100,100') to be positioned between the piston rod (220) and the piston (214) in accordance with said size of axial clearance,
- modifying the axial dimension (H) of the piston washer assembly (100,100') by moving the proximal part (110, 110') and the distal part (120, 120') relative to each other to increase said axial dimension (H) until the proximal part (110, 110') and the distal part (120, 120') assume a target state where said axial dimension (H) reaches an axial target dimension (H2), and
- mutually interconnecting the first sub-assembly (100a) and the second sub-assembly (100b) with the piston washer assembly (100,100') positioned between the piston rod (220) and the piston (214).

## Patentansprüche

1. Kolbendichtungsanordnung zur Verwendung in einer Arzneimittelabgabevorrichtung zum Übertragen einer distal gerichteten Axialkraft von einer Kolbenstange in Richtung eines Kolbens einer gehaltenen Kartusche, die Kolbendichtungsanordnung (100, 100') umfassend:
- einen proximalen Teil (110, 110'), der zum Eingriff mit dem distalen Abschnitt einer Kolbenstange (220) ausgelegt ist, wobei der proximale Teil (110, 110') einen ersten Satz von zusammenwirkenden Geometrien (115, 115') definiert, und
- einen distalen Teil (120, 120'), der zum Eingriff mit und Anliegen an einem Kolben (214) einer gehaltenen Kartusche (213) und zum Eingriff mit dem proximalen Teil (110, 110') konfiguriert ist, wobei der distale Teil (120, 120') einen zweiten Satz von zusammenwirkenden Geometrien (125, 125') definiert, die den ersten Satz von zusammenwirkenden Geometrien (115, 115') in Eingriff nehmen,
wobei, in einem Anfangszustand der Anordnung, der proximale Teil (110, 110') und der distale Teil (120, 120') relativ zueinander positioniert sind, um eine anfängliche axiale Abmessung (H, H1) für die Anordnung zu definieren, und wobei der proximale Teil (110, 110') und der distale Teil (120, 120') relativ zueinander beweglich sind durch Bewegen des ersten Satzes von zusammenwirkenden Geometrien (115, 115') und des zweiten Satzes von zusammenwirkenden Geometrien (125, 125') relativ zueinander, um die axiale Abmessung (H) zu vergrößern, bis der proximale Teil (110, 110') und der distale Teil (120, 120') einen Zielzustand annehmen, in dem die axiale Abmessung (H) eine axiale Zielabmessung (H2) erreicht, und
wobei der erste Satz von zusammenwirkenden Geometrien (115, 115') und der zweite Satz von zusammenwirkenden Geometrien (125, 125') konfiguriert sind, zu verhindern, dass der distale Teil (120, 120') und der proximale Teil (110, 110') relativ zueinander bewegt werden, wenn Kräfte auf den proximalen Teil (110, 110') und den distalen Teil (120, 120') wirken, um die axiale Zielabmessung (H2) zu verkleinern.

2. Kolbendichtungsanordnung nach Anspruch 1, wobei der erste Satz von zusammenwirkenden Geometrien (115, 115') und der zweite Satz von zusammenwirkenden Geometrien (125, 125') konfiguriert sind, einen Rastmechanismus (115a, 125a) bereitzustellen, der ein Vergrößern der axialen Abmessung (H) zulässt und ein Verkleinern der axialen Abmessung (H) gleichzeitig verhindert.

3. Kolbendichtungsanordnung nach einem der Ansprüche 1-2, wobei der erste Satz von zusammenwirkenden Geometrien (115, 115') und der zweite Satz von zusammenwirkenden Geometrien (125, 125') einen schrittweisen inkrementellen Einweg-Rastmechanismus definieren, der ein Vergrößern der axialen Abmessung (H) in Inkrementalschritten der Einheitsgröße ΔH zulässt, und wobei, wenn der proximale Teil (110, 110') und der distale Teil (120, 120') den Zielzustand annehmen, der erste Satz von zusammenwirkenden Geometrien (115, 115') und der zweite Satz von zusammenwirkenden Geometrien (125, 125') eine Bewegung, in einer Größe ΔH, des ersten Teils und des zweiten Teils relativ zueinander, die so wirkt, dass die axiale Abmessung (H) verkleinert wird, verhindern.

4. Kolbendichtungsanordnung nach einem der Ansprüche 1-3, wobei der erste Satz von zusammenwirkenden Geometrien (115') eine erste Gewindekonfiguration definiert und der zweite Satz von zusammenwirkenden Geometrien (125') eine zweite Gewindekonfiguration, die die erste Gewindekonfiguration in Eingriff nimmt, definiert, und wobei eine relative Drehbewegung in einer ersten Drehrichtung zwischen dem proximalen Teil (110') und dem distalen Teil (120') die axiale Abmessung (H) vergrößert.

5. Kolbendichtungsanordnung nach Anspruch 4, wobei ein Mechanismus inkrementeller Positionierung zwischen dem proximalen Teil (110') und dem distalen Teil (120') angeordnet ist, der bewirkt, dass die relative Drehbewegung zwischen dem proximalen Teil (110, 110') und dem distalen Teil (120') in inkrementellen Winkelschritten erfolgt.

6. Kolbendichtungsanordnung nach Anspruch 5, wobei der Mechanismus inkrementeller Positionierung als zusammenwirkende Rastgeometrien, die in der ersten Gewindekonfiguration (115') und der zweiten Gewindekonfiguration (125') angeordnet sind, bereitgestellt ist.

7. Kolbendichtungsanordnung nach einem der Ansprüche 1-3, wobei der erste Satz von zusammenwirkenden Geometrien (115) und der zweite Satz von zusammenwirkenden Geometrien (125) eine Linearführung definieren, die eine lineare axiale Bewegung zwischen dem proximalen Teil (110) und dem distalen Teil (120) ermöglicht, und wobei der erste Satz von zusammenwirkenden Geometrien (115) und der zweite Satz von zusammenwirkenden Geometrien (125) einen Einweg-Klinken-Zahneingriff definieren, der ein Vergrößern der axialen Abmessung (H) zulässt, ein Verkleinern der axialen Abmessung (H) jedoch verhindert.

8. Kolbendichtungsanordnung nach einem der Ansprüche 1-7, wobei die Kolbendichtungsanordnung (100, 100') einen Umfangsabschnitt mit einem Durchmesser einer ersten Größe definiert, und wobei die maximal einstellbare axiale Abmessung (H) kleiner ist als die erste Größe.

9. Arzneimittelabgabevorrichtung zum Ausstoßen einer Dosis eines Arzneimittels aus einer gehaltenen Kartusche, wobei die Arzneimittelabgabevorrichtung ein distales Arzneimittel-Auslassende und ein gegenüberliegendes proximales Ende definiert, und umfassend:
- eine Gehäusekomponente (210), die eine Kartusche (213) hält, die ein flüssiges Arzneimittel und einen Kolben (214), der darin in einer axialen Richtung verschiebbar angeordnet ist, umfasst,
- einen Dosiseinstellungs- und Ausstoßmechanismus, der mit der Gehäusekomponente (210) gekoppelt ist und eine Kolbenstange (220) zum Ausüben einer Kraft auf den Kolben (214) der Kartusche (213) in einer distalen Richtung zum Ausstoßen einer Dosis umfasst, und
wobei eine Kolbendichtungsanordnung (100, 100') nach einem der Ansprüche 1-8 axial zwischen dem Kolben (214) der Kartusche (213) und der Kolbenstange (220) angeordnet ist.

10. Arzneimittelabgabevorrichtung nach Anspruch 9, wobei sich die Kolbenstange (220) während des Ausstoßens der Dosis dreht, und wobei die Kolbenstange (220) relativ zur Kolbendichtungsanordnung (100, 100') drehbar angeordnet ist, um der Kolbenstange (220) ein Drehen zu ermöglichen, während die Kolbendichtungsanordnung (100, 100') weiterhin nicht drehbar bleibt.

11. Verfahren zur Herstellung einer Kolbendichtungsanordnung (100, 100') nach einem der Ansprüche 1-10 zur Verwendung in einer Arzneimittelabgabevorrichtung zum Übertragen einer distal gerichteten Axialkraft von einer Kolbenstange in Richtung eines Kolbens einer gehaltenen Kartusche, umfassend die folgenden Schritte:
- Bereitstellen des proximalen Teils (110, 110') und des distalen Teils (120, 120') in einem anfänglichen Eingriffszustand, wobei der proximale Teil (110, 110') und der distale Teil (120, 120') relativ zueinander positioniert sind, um eine anfängliche axiale Abmessung (H, H1) für die Kolbendichtungsanordnung (100, 100') zu definieren,
- Schätzen einer gewünschten axialen Zielabmessung (H2) für die Kolbendichtungsanordnung (100, 100'), und
- Bewegen des proximalen Teils (110, 110') und des distalen Teils (120, 120') relativ zueinander, um die axiale Abmessung (H) zu vergrößern, bis der proximale Teil (110, 110') und der distale Teil (120, 120') einen Zielzustand annehmen, in dem die axiale Abmessung (H) die gewünschte axiale Zielabmessung (H2) erreicht.

12. Verfahren zur Herstellung einer Kolbendichtungsanordnung nach Anspruch 11, umfassend die folgenden Schritte:
- Bereitstellen eines von dem distalen Teil (120, 120') und dem proximalen Teil (110, 110') als erster Einspritzschuss in einem Mehrschuss-Spritzgießprozess, und
- Bereitstellen des anderen von dem distalen Teil (120, 120') und dem proximalen Teil (110, 110') als weiterer Einspritzschuss in einem Mehrschuss-Spritzgießprozess, sodass der erste Satz von zusammenwirkenden Geometrien (115, 115') und der zweite Satz von zusammenwirkenden Geometrien (125, 125') zumindest teilweise in Eingriff sind.

13. Verfahren zur Herstellung einer Kolbendichtungsanordnung nach einem der Ansprüche 11-12, wobei der Schritt des Bewegens des proximalen Teils (110, 110') und des distalen Teils (120, 120') relativ zueinander, um die axiale Abmessung (H) zu vergrößern, bis der proximale Teil (110, 110') und der distale Teil einen Zielzustand annehmen, in dem die axiale Abmessung (H) die gewünschte axiale Zielabmessung (H2) erreicht, die folgenden Schritte umfasst:
- Ineingriffhalten des distalen Teils (120, 120') mit einem ersten Werkzeugteil,
- Ineingriffhalten des proximalen Teils (110, 110') mit einem zweiten Werkzeugteil,
- Bewegen des ersten Werkzeugteils und des zweiten Werkzeugteils relativ zueinander, während die Größe der axialen Abmessung (H) der Kolbendichtungsanordnung (100, 100') gemessen, ermittelt oder geschätzt wird.

14. Verfahren zum Montieren einer Arzneimittelabgabevorrichtung nach einem der Ansprüche 9-10, umfassend die folgenden Schritte:
- Positionieren der Kartusche (213), die ein flüssiges Arzneimittel und einen verschiebbar darin angeordneten Kolben (214) umfasst, in einer ersten Gehäusekomponente (210), um eine erste Unteranordnung (100a) zu bilden,
- Positionieren des Dosiseinstellungs- und Ausstoßmechanismus, der die Kolbenstange (220) umfasst, in einer zweiten Gehäusekomponente (201), um eine zweite Unteranordnung (100b) zu bilden,
- Ermitteln der axialen Position des Kolbens (214) in Bezug auf die erste Gehäusekomponente (210),
- Ermitteln der axialen Position der Kolbenstange (220) in Bezug auf die zweite Gehäusekomponente (201),
- Ermitteln oder Schätzen der Größe des Axialspiels zwischen der Kolbenstange (220) und dem Kolben (213), sofern die erste Unteranordnung (100a) und die zweite Unteranordnung (100b) montiert wären, und Auswählen einer axialen Zielabmessung (H2) für eine Kolbendichtungsanordnung (100, 100'), die zwischen der Kolbenstange (220) und dem Kolben (214) positioniert werden soll, gemäß der Größe des Axialspiels,
- Modifizieren der axialen Abmessung (H) der Kolbendichtungsanordnung (100, 100') durch Bewegen des proximalen Teils (110, 110') und des distalen Teils (120, 120') relativ zueinander, um die axiale Abmessung (H) zu vergrößern, bis der proximale Teil (110, 110') und der distale Teil (120, 120') einen Zielzustand annehmen, in dem die axiale Abmessung (H) eine axiale Zielabmessung (H2) erreicht, und
- gegenseitiges Verbinden von der ersten Unteranordnung (100a) und der zweiten Unteranordnung (100b) mit der Kolbendichtungsanordnung (100, 100'), die zwischen der Kolbenstange (220) und dem Kolben (214) positioniert ist.

## Revendications

1. Ensemble de rondelle de piston pour l'utilisation dans un dispositif d'administration de médicaments pour le transfert d'une force axiale dirigée distalement d'une tige de piston vers un piston d'une cartouche tenue, l'ensemble de rondelle de piston (100, 100') comprenant :
- une partie proximale (110, 110') adaptée pour l'engagement avec la partie distale d'une tige de piston (220), la partie proximale (110, 110') définissant un premier ensemble de géométries coopérantes (115,115'), et
- une partie distale (120, 120') configurée pour engager et abouter un piston (214) d'une cartouche tenue (213) et pour engager la partie proximale (110, 110'), où la partie distale (120, 120') définit un deuxième ensemble de géométries coopérantes (125, 125') engageant le premier ensemble de géométries coopérantes (115, 115'),
où, dans un état initial de l'assemblage, la partie proximale (110, 110') et la partie distale (120, 120') sont positionnées relativement l'une l'autre pour définir une dimension axiale initiale (H, H1) pour l'assemblage, et où la partie proximale (110, 110') et la partie distale (120, 120') peuuvent être déplacées l'une par rapport à l'autre en déplaçant le premier ensemble de géométries coopérantes (115, 115') et le deuxième ensemble de géométries coopérantes (125, 125') les unes par rapport aux autres pour augmenter ladite dimension axiale (H) jusqu'à ce que la partie proximale (110, 110') et la partie distale (120, 120') assument un état cible dans lequel ladite dimension axiale (H) atteint une dimension axiale cible (H2), et
où le premier ensemble de géométries coopérantes (115, 115') et le deuxième ensemble de géométries coopérantes (125, 125') sont configurés pour empêcher la partie distale (120, 120') et la partie proximale (110, 110') d'être déplacée l'une par rapport à l'autre lorsque les forces agissent sur la partie proximale (110, 110') et la partie distale (120, 120') pour diminuer ladite dimension axiale cible (H2).

2. Ensemble de rondelle de piston selon la revendication 1, dans lequel le premier ensemble de géométries coopérantes (115, 115') et le deuxième ensemble de géométries coopérantes (125, 125') sont configurés pour fournir un mécanisme de détente (115a, 125a) permettant à la dimension axiale (H) d'être augmentée tout en empêchant la dimension axiale (H) d'être diminuée.

3. Ensemble de rondelle de piston selon l'une quelconque des revendications 1 à 2, dans lequel le premier ensemble de géométries coopérantes (115, 115') et le deuxième ensemble de géométries coopérantes (125, 125') définissent un mécanisme de détente incrémentiel à sens unique permettant à la dimension axiale (H) d'être augmentée en étapes incrémentielles de la taille unitaire ΔH, et dans lequel, lorsque la partie proximale (110, 110') et la partie distale (120, 120') assument l'état cible, le premier ensemble de géométries coopérantes (115, 115') et le deuxième ensemble de géométries de coopérantes (125, 125') empêchent le mouvement d'amplitude ΔH de la première partie et de la deuxième partie l'une par rapport à l'autre agissant pour réduire la dimension axiale (H).

4. Ensemble de rondelle de piston selon l'une quelconque des revendications 1 à 3, dans lequel le premier ensemble de géométries coopérantes (115') définit une première configuration de filetage et le deuxième ensemble de géométries coopérantes (125') définit une deuxième configuration de filetage engageant la première configuration de filetage, et dans lequel un mouvement de rotation relatif dans une première direction de rotation entre la partie proximale (110') et la partie distale (120') augmente la dimension axiale (H).

5. Ensemble de rondelle de piston selon la revendication 4, dans lequel un mécanisme de positionnement incrémentiel est disposé entre la partie proximale (110') et la partie distale (120') amenant ledit mouvement de rotation relatif entre la partie proximale (110, 110') et la partie distale (120') à se produire en étapes angulaires incrémentielles.

6. Ensemble de rondelle de piston selon la revendication 5, dans lequel ledit mécanisme de positionnement incrémentiel est fourni sous forme de géométries de détente coopérantes disposées dans la première configuration de filetage (115') et la deuxième configuration de filetage (125').

7. Ensemble de rondelle de piston selon l'une quelconque des revendications 1 à 3, dans lequel le premier ensemble de géométries coopérantes (115) et le deuxième ensemble de géométries coopérantes (125) définissent un guide linéaire permettant un mouvement axial linéaire entre la partie proximale (110) et la partie distale (120) et dans lequel le premier ensemble de géométries coopérantes (115) et le deuxième ensemble de géométries coopérantes (125) définissant un engagement à cliquet à sens unique denté permettant à la dimension axiale (H) d'être augmentée, mais empêchant la dimension axiale (H) d'être diminuée.

8. Ensemble de rondelle de piston selon l'une quelconque des revendications 1 à 7, dans lequel l'ensemble de rondelle de piston (100, 100') définit une section périphérique ayant un diamètre d'une première magnitude et dans lequel la dimension axiale (H) réglable maximale est inférieure à la première amplitude.

9. Dispositif d'administration de médicaments pour l'expulsion d'une dose de médicament d'une cartouche tenue, le dispositif d'administration de médicament définissant une extrémité distale de sortie de médicament et une extrémité proximale opposée et comprenant :
- un composant du boîtier (210) tenant une cartouche (213) comprenant un médicament liquide et un piston (214) disposé dans celle-ci de manière coulissante dans une direction axiale,
- un mécanisme de réglage et d'expulsion de dose couplé au composant du boîtier (210) et comprenant une tige de piston (220) pour l'exercice d'une force sur le piston (214) de la cartouche (213) dans une direction distale pour l'expulsion d'une dose, et
où un ensemble de rondelle de piston (100, 100') selon l'une quelconque des revendications 1-8 est disposé axialement entre le piston (214) de la cartouche (213) et la tige de piston (220).

10. Dispositif d'administration de médicament selon la revendication 9, dans lequel la tige de piston (220) tourne pendant l'expulsion de la dose et dans lequel la tige de piston (220) est disposée de manière rotative par rapport à l'ensemble de rondelle de piston (100, 100') pour permettre à la tige du piston (220) de tourner alors que l'ensemble de rondelle de piston (100, 100') reste non rotatif.

11. Procédé de préparation d'un assemblage de rondelle de piston (100, 100') tel que défini dans l'une quelconque des revendications 1 à 10 pour l'utilisation dans un dispositif d'administration de médicament pour le transfert d'une force axiale dirigée distalement d'une tige de piston vers un piston d'une cartouche tenue, comprenant les étapes de :
- la fourniture de la partie proximale (110, 110') et de la partie distale (120, 120') dans un état initialement engagé, où la partie proximale (110, 110') et la partie distale (120, 120') sont positionnées relativement l'un l'autre pour définir une dimension axiale initiale (H, H1) pour l'ensemble de rondelle de piston (100, 100'),
- l'estimation d'une dimension axiale cible souhaitée (H2) pour l'ensemble de rondelle de piston (100, 100'), et
- le déplacement de la partie proximale (110, 110') et de la partie distale (120, 120') l'une par rapport à l'autre pour augmenter ladite dimension axiale (H) jusqu'à ce que la partie proximale (110, 110') et la partie distale (120, 120') assument un état cible où ladite dimension axiale (H) atteint la dimension axiale cible souhaitée (H2).

12. Procédé de préparation d'un assemblage de rondelle de piston selon la revendication 11, comprenant les étapes de :
- la fourniture de l'une de la partie distale (120, 120') et de la partie proximale (110, 110') comme première injection dans un procédé de moulage par injection multi-injections, et
- la fourniture de l'autre de la partie distale (120, 120') et de la partie proximale (110, 110') comme une injection supplémentaire dans un procédé de moulage par injection multi-injection de sorte que le premier ensemble de géométries coopérantes (115, 115') et le deuxième ensemble de géométries coopérantes (125, 125') s'engagent au moins partiellement.

13. Procédé de préparation d'un ensemble de rondelle de piston selon l'une quelconque des revendications 11 à 12, dans lequel l'étape de déplacement de la partie proximale (110, 110') et de la partie distale (120, 120') l'une par rapport à l'autre pour augmenter ladite dimension axiale (H) jusqu'à ce que la partie proximale (110, 110') et la partie distale assument un état cible où ladite dimension axiale (H) atteint une dimension axiale cible (H2) comprenant les étapes du :
- maintien de la partie distale (120, 120') engagée avec une première pièce d'outil,
- maintien de la partie proximale (110, 110') engagée avec une deuxième pièce d'outil,
- déplacement de la première partie d'outil et de la deuxième partie d'outil l'une par rapport à l'autre lors de la mesure, de la détermination ou de l'estimation de la taille de ladite dimension axiale (H) de l'ensemble de rondelle de piston (100, 100').

14. Procédé d'assemblage d'un dispositif d'administration de médicament selon l'une quelconque des revendications 9 à 10, comprenant les étapes :
- du positionnement de la cartouche (213) comprenant un médicament liquide et d'un piston (214) disposés dans celle-ci de manière coulissante dans un premier composant du boîtier (210) pour former un premier sous-ensemble (100a),
- du positionnement du mécanisme de églage et d'expulsion comprenant la tige de piston (220) dans un deuxième composant du boîtier (201) pour former un deuxième sous-ensemble (100b),
- de la détermination de la position axiale du piston (214) par rapport au premier composant du boîtier (210),
- de la détermination de la position axiale de la tige de piston (220) par rapport au deuxième composant du boîtier (201),
- de la détermination ou de l'estimation de la taille du dégagement axial entre la tige de piston (220) et le piston (213) si le premier sous-ensemble (100a) et le deuxième sous-ensemble (100b) ont été assemblés et la sélection d'une dimension axiale cible (H2) pour un assemblage de rondelle de piston (100, 100') à positionner entre la tige de piston (220) et le piston (214) conformément à ladite taille de dégagement axial,
- de la modification de la dimension axiale (H) de l'ensemble de rondelle de piston (100, 100') en déplaçant la partie proximale (110, 110') et la partie distale (120, 120') l'une par rapport à l'autre pour augmenter ladite dimension axiale (H) jusqu'à ce que la partie proximale (110, 110') et la partie distale (120, 120') assument un état cible où ladite dimension axiale (H) atteint une dimension axiale cible (H2), et
- de l'interconnexion mutuelle du premier sous-ensemble (100a) et du deuxième sous-ensemble (100b) avec l'ensemble de rondelle de piston (100, 100') positionné entre la tige de piston (220) et le piston (214).
